# EUROPEAN PATENT APPLICATION

(11) **EP 1 600 185 A1**
(43) Date of publication of application: **30.11.2005**
(21) Application number: 05011210.1
(22) Date of filing: 24.05.2005
(51) Int. Cl.: A61M 3/02, A61M 15/00

(54) **Vaginal spray nozzle**

(30) Priority: 28.05.2004 IT BO20040338
(71) Applicant: Progine Farmaceutici Srl, 50041 Calenzano (Florence) (IT)
(72) Inventor: Landi, Lapo, 50019 Sesto Fiorentino (Florence) (IT)
(74) Representative: Paolini, Elena

(57) **Abstract**

A container (1) with an upper threaded part (2) is provided to which is screwed a suction pump (3) with inside thread (4). Said pump with a cylindrical outside body (5) and with a lower pipe (6) to suck the liquid contained in the container (1). Said pipe (6) is fitted into a valve (7) having in the upper part a push-rod (11) with piston (12) in contact with the main spring (13) and having in its inside a stem (14) that goes to fit into the cylindrical concavity (16) with a hole (17) communicates with a cannula (18) having on its end a hole (20) and placed onto the outside part of a cylindrical body (19).

## Description

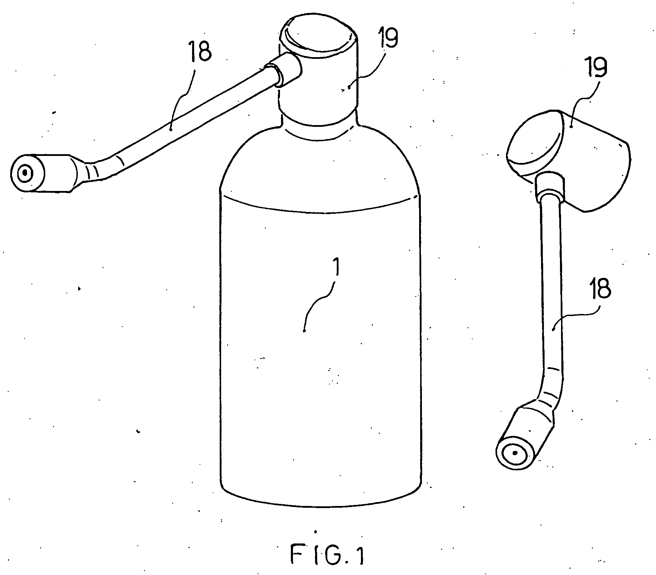

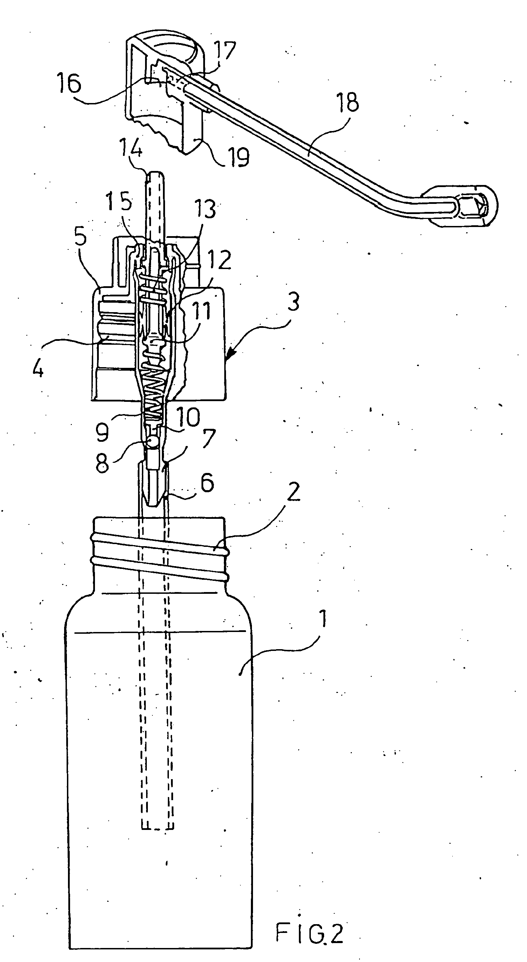

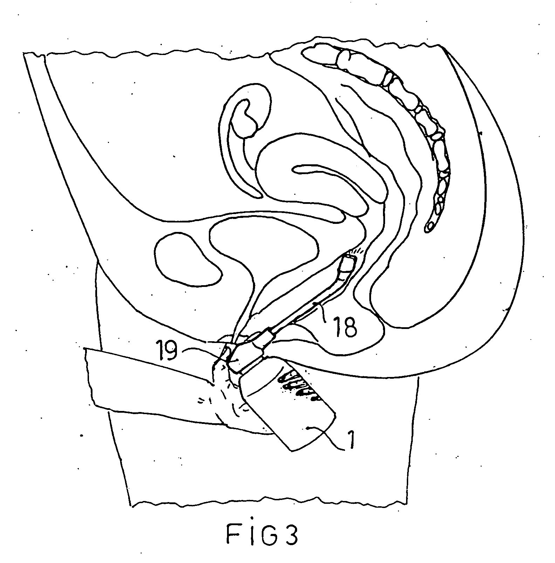

Within the gynecology has big importance the getting in of pharmaceutical substances and/or detergents inside the vagina. The distribution of said substances is of fundamental importance both for the absorption to the vaginal tissues than for the permanence of the active factor of the medicinal substance in the parts with inflammations or lesions. In the art the medical substances and/or detergents are put inside the vagina by means of lavages, foams or ovules. The vaginal lavages consists of liquids inside containers with cannulas. Squeezing the container walls the liquid runs off. Said liquid goes out to the cannula to flow inside the vagina and then it flows outside. Unfortunately this kind of use has the risk of contamination upward. Infact, said liquid, running upward, brings a diffusion of bacteria, cells or others microorganisms to the uterus and to the cervix uteri. Moreover, this application way brings the active factors of the medicament in contact with the inflamed or ill tissue for short time and it is little persistent. Other method provides the application of vaginal foams. These are spray foams with compressed gas into a bombs from which get out liquid or foam. Unfortunately also this method has sides that limit its uses. Infact, the gas used like propellant is also it an irritation cause. While the methods that use ecological foams without propellant gas have only the deterging function. In these cases, infact, the foam gets out and, turning into water solution, it percolates to the part in which was put so being, in this way, troublesome. Moreover, this kind of use has a short contact time onto the irritated or infect part. Other kind of application of the medicaments is actuated by means of vaginal ovules. These ovules are introduced in the vagina with a finger, but so introducing also bacteria. The vaginal ovules have a longer time of application, such as they remain in position for some hours, but, being formed of excipients like semisynthetic triglycerides and thickenings or similar substances, they leaves residuals so to have the necessity to do vaginal lavages after that said ovules are dissolved. The used excipients and thickenings have, moreover, the problem to often create allergies to the vaginal walls. Moreover the ovule, such as it is very concentrated in the active factor, determines heartburns in the point where it is positioned. Similar problems are given to the creams or to the vaginal gels, sometimes used in single-dose to be applied with tubes and syringes, because they have creams and excipients that leave inside fat residuals. The invented means, different to the previous art, consists of a spray nozzle that permits to reach the critical points of the vagina without manual contact, actuating a nebulization of the medical and/or deterging substance. In this way a uniform distribution to the vaginal walls is obtained helping to the presence in position of gel with low viscosity or liquids in solution put in circulation to the nozzle spray. In this way good adherence characteristics are obtained with consequent longer contact time of the lavages or of the foams with the parts to be treated. The getting in is, then, actuated with a jet having the least pressure so to not go upward the bacteria or the vaginal secretion residuals so avoiding to help vaginal erosions. The invented means, infact, reaches directly the cervix uteri acting onto the parts interested to lesions or inflammations. Moreover, different to the ovule that concentrates the substance effect in one point, the invented means permits to distribute the active substance long all the vaginal duct, starting to the top and in succession descending, with an uniform and persistent distribution onto the vaginal walls. The invented spray nozzle consists of a container 1 with the upper part having a thread 2. In said container 1 is threaded, through the upper thread 2, a suction pump 3. Said pump has an inside thread 4, complementary to the previous one, and it has an outside cylindrical body 5. The suction pump 3 has in the lower part a pipe 6 that goes to suck the fluid inside the container 1. Said pipe 6 is then fitted in the cylindrical body of a valve 7. Inside said valve is put a sphere 8 pressing against the inside walls of the valve by means of a spring 9 and of a holding means 10. In the upper part and in the middle of the spring 9 is present a push-rod 11 with, in standstill to in the upper part, a piston 12. Said piston is itself in contact with the main spring 13. Inside said piston, coaxially, is provided a stem 14 inside which runs the upper cylindrical part of the push-rod 11.To contain the valve 7 there is a flange 15 fitted in onto the inside part of the cylindrical body 5. The jutted out part of the stem 14 is fitted into the cylindrical concavity 16 with a hole 17 communicates with the cannula 18 placed onto the outside part of the cylindrical body 19. Said cannula 18 has a length so to can easy reach the cervix uteri. The cannula 18 has on its end a hole 20 from where the contained liquid is nebulized. The end of said cannula is in ogive shape. Squeezing in the upper part the cylindrical body 19, and consequently the stem 14, the substance present inside the container 1 is nebulized. The invented means is, moreover, equipped of changing parts to be used only one time to not bring, through the cannula 18, again the bacteria into the vagina. For this reason the upper part fitted in onto the stem 14 is to be changed after each use. Said part, i.e. the upper cylindrical body 19 and the cannula 18, in non-toxic plastic material, is the part that is introduced into the vagina and so can be subjected to bacteria , virus, cells and other formations. Moreover, being the nebulizaton with at least pressure but sufficient to permit it , does not bring into the vagina neither what previous cited nor neoplastic cells. The invented means, moreover, permits to increase the contact time of the active medical substances and to have an uniform distribution long all the vaginal duct such as, moving the spray nozzle during the use, there is an uniform and persistent distribution onto the walls. The invented means is illustrated in a merely indicative and not limiting way in the drawings of sheets 1, 2 and 3. In sheet 1 figure 1 is perspective view of the vaginal spray nozzle with the part to be changed in the subsequent use. In sheet 2 the figure 2 is exploded view to the nozzle. In sheet 3 figure 2 is view of the use with the medical substance puts into the vagina by means of the invented spray nozzle.

## Claims

1. Vaginal spray nozzle **characterized in that** to consist of a container (1) with an upper threaded part (2) to which is screwed a suction pump (3) with inside thread (4), with cylindrical outside body (5) and with a lower pipe (6) to suck the liquid contained in the container (1); and with said pipe (6) fitted into a valve (7) having in the upper part a push-rod (11) with piston (12) in contact with the main spring (13) and having in its inside a stem (14) that goes to fit into the cylindrical concavity (16) with a hole (17) communicates with a cannula (18) having on its end a hole (20) and placed onto the outside part of a cylindrical body (19).

2. Vaginal spray nozzle, as per claim 1, **characterized in that** the cannula (18) has a length so to easy reach the cervix uteri.

3. Vaginal spray nozzle, as per claim 1, **characterized in that** the hole (20) at the end of the cannula (18) permits to nebulization of the liquid present in the container (1).

4. Vaginal spray nozzle, as per claim 1, **characterized in that** squeezing in the upper part the cylindrical body (19) and consequently the stem (14), the substance present inside the container (1) is nebulized.

5. Vaginal spray nozzle, as per claim 1, **characterized in that** the upper cylindrical body (19) and the cannula (18), in non-toxic plastic material, are to be changed after each use to avoid infections.

6. Vaginal spray nozzle, as per claim 1, **characterized in that** it permits to increase the contact time of the medical substances and to have an uniform distribution long all the vaginal duct.

7. Vaginal spray nozzle, as per claim 1, **characterized in that** being the nebulization with least pressure, but sufficient to permit it, does not bring into the vagina the bacteria, virus or other.
